# EUROPÄISCHE PATENTANMELDUNG

(11) **EP 0 835 665 A2**
(43) Veröffentlichungstag der Anmeldung: **15.04.1998**
(21) Anmeldenummer: 97117577.3
(22) Anmeldetag: 10.10.1997
(51) Int. Cl.: A61L 2/24, A61L 2/18, A61B 1/00

(54) **Desinfektionsautomat zur Reinigung, Desinfektion, Neutralisation und Trocknung von medizinischen Aufbereitungsgut**

(30) Priorität: 10.10.1996 DE 19641738
(71) Anmelder: Krause, Irene, 30966 Arnum (DE)
(72) Erfinder: Krause, Irene, 30966 Arnum (DE)
(74) Vertreter: Patentanwälte Thömen & Körner

(57) **Zusammenfassung**

Es wird eine Vorrichtung zur Desinfektion insbesondere von medizinischen Apparaten und Geräten offenbart, welche aus einer Kammer besteht, in welche die zu reinigenden Geräte aufgehängt werden können und in welche ein chemisches Desinfektionsmittel einspeisbar ist. Erfindungsgemäß ist vorgesehen, daß das zu reinigende medizinische Gerät senkrecht über einer Geräteaufnahme positioniert wird, wobei eine variable Höhenverstellung manuell, durch einen Elektrogetriebemotor oder auf pneumatischem Wege für ein arbeitsökonomisches einfaches Handling der Beschickung und der Entnahme des zu reinigenden Gerätes sorgt.

## Beschreibung

### 1. Technisches Gebiet: Anwendungsbereich

Das vollautomatische Aufbereitungsverfahren zur Reinigung, Desinfektion, Neutralisation und Trocknung von medizinischem Waschgut, hat die Aufgabe durch einen wählbaren standardisiertem Ablauf im geschlossenen System der Aufbereitungskammern eine einwandfreie hygienische Desinfektion zu gewährleisten.

Der Anwendungsbereich der Erfindung ist besonders der Einsatzbereich von medizinisch thermolabilem Material, das heißt;
Temperaturbereich in der Reinigung, Desinfektion und Neutralisation bis maximal 60° C .
Dieser Temperaturbereich wird als themolabil bezeichnet, da Kunststoffmaterial und Klebeverbindungsstellen in den Fließbereich kommen. Diese Anwendung erfolgt bei Instrumenten, Schläuchen und Geräten sowie für Kombinationsinstrumente aus Metall und Kunststoff sowie für anderes OP- und Klinikwaschgut.

Als Fallbeispiel für die Beschreibung der Erfindung ist die Anwendung, das hygienische vollautomastische Aufbereitungsverfahren für Einsatzbereich flexibele Endoskope, dargestellt.
Das flexible Endoskop in der Bauart, als operatives Untersuchungsinstrument für oberen oder unteren Verdauungstrakt, auf die Funktions- und den Konstruktionstyp, sowie auf die Art der Bildübertragung des flexiblen Endoskopes über Glasfaser oder durch Videochip bleibt nachfolgend unberücksichtigt.

Anwendbar ist die Erfindernutzung sowohl in der Humanmedizin, der Veterinärmedizin und für Industrieanwendungen.
Die nachstehende Nennung von Aufarbeitungsgut erhebt keinen Anspruch auf Vollständigkeit der Nutzungsmöglichkeiten sondern öffnet nur einen Ausblick, auf die Anwendungsmöglichkeiten.

Als Aufbereitungsgut wird nachstehend genannt;

Gastroskope, Duodenoskope, Koloskope, Sigmoidoskope, Choledochoskope, Bronchoskope, Nasopharingoskope, Hysteroskope, Laparoskope und Atros-Nephroskope.
Weitere Sonden aller Art zum Einführen in Körperöffnungen natürlicher oder chirurgischer Art. Wie Transesophogeal Ultrasonic Instrumente und Einführungssonden mit Bilderkennung auf dem Übertragungswege Ultraschall oder Sonographisch. Desweiteren OP-Instrumente, zur Diagnose und Therapie, MIC-Instrumente, Fremdkörperzangen, Biopsiezangen, Spülkatheter, Zytologiebürsten, Koagulationszangen - und Elektroden, Hot-Biopsie Zangen, Diathermie-Schlingen, Papillotome und anderes Instrumentarium mehr.
Die weitere große Anwendungsgruppe betrifft die Beatmungs-und Nakoseschläuche, sowie Schlauchmaterial für Pharmazeutik, Blut, Infusionen und Dialyse.

Neben dem Einsatzbereich für thermolabiles Aufbereitungsgut, ist die Erfindung, durch eine Doppelmantelkonstruktion mit Isolierung als Option auch für den chemotermischem vollautomatischen Aufbereitung bis ca. 100°C maximal einsetzbar. Die gleiche erfindergemäße Konstruktionsart ist folglich auch für höhere Temparaturbereiche verwendbar, hierzu ist eine Temperaturregelung erforderlich.

Die vollautomatischen Aufbereitungsverfahren unterscheiden sich in den Temparaturbereichen bis C 60°. Hier erfolgt eine Wiederverwendung des Desinfektionsmittels. Bei der chemotermischen Warmdesinfektion wird in dem automatischen Reinigungs-und Desinfektionsablauf in jeder Charge, die verwendeten Reinungs-Desinfektionsmittel nach dem programmierten Programmablauf dem Abwasser zugeführt. Es erfolgt keine Wiederverwendung.

### 1.11 Allgemeiner Technischer Stand

Die derzeit auf dem Markt befindlichen automatischen, chemischen und chemothermisch arbeitenden vollautomatischen Reinigungs-und Desinfektionsmaschinen zeichnen sich zwar durch einen standardisierten Ablauf, geringen Personal-und Zeitaufwand aus, haben jedoch einen hohen Energie-, Desinfektionsmittel- und Wasserverbrauch.

Desweiteren verbesserungswürdig ist jedoch die hygienische Qualität des gereinigten und desinfizierten Spülgutes.

Als Ursache dieser Mängel ist die spültechnisch ungünstige, waagerechte, leicht nach unten geführte Kreislage des Waschgutes auf den Halterungen der Beschickungskörbe anzusehen. An allen Auflagepunkten des Waschgutes, Endoskop, setzen sich Schmutzpartikel durch die Düsenwaschungen fest. Die Verunreinigungsreste haben durch den Einschluß von Mikroorganismen sowie durch eine schnelle Verkrustung und Abkapselung im Kernbereich Vieren, die den Desinfektionsprozeß ohne Abtötung überstehen können. Bei der Entnahme der Instrumente, kommt es durch Bedienungshändling zur Kontamienierung der Außenflächen der Instrumente.

Als Verursachungszone ist die kreisförmige, zum Teil übereinander angeordnete Positionierung und Auflage der flexiblen Endoskope zu sehen. In der Kreislage entstehen in dem dünnwandigen Kunststoff- Gummimaterial, Knickstellen und Faltentaschen sowohl auf der Außenseite, wie in den engen dünnwandigen Innenkanälen die in den flexiblen Teilen aus einem mehrlagien Material gefertigt sind.
Durch diese Faltentaschen wird ein schneller Flüssigkeisablauf verhindert. Durch die Knickstellen kommt es zu Spülschatten, diese führen zu einer schlechten Reinigungs- und Desinfektionsqualität des Instrumentarium.

Die weitere Gefahr einer Übertragung von gefährlichen Krankheitskeimen besteht in der Hinsicht, das sich in den Knick-und Faltentaschen Rückstände des Reinigungs- und Desinfektionsmittel sammeln, die durch die nachfolgende Neutralisation nicht genügend herausgespült werden und die dann durch die Trocknung pulverisieren. Durch die häufig poröse, rauhere Materialbeschaffenheit in den Knickstellen betten sich die pulverisierten Rückstände in das Kunststoff und Schlauchmaterial ein. Bei den flexiblen Endoskopen ist der Außenmantel, der Einführungsteil zusätzlich mit einer Schutzglanzschicht versehen, durch Nutzung der Instrumente kommt es zu kleinen Rißbildungen in den häufig beanspruchten Biegestellen der Oberflächenschicht zu Ansammlungen von möglichen kritischen Bakterien.
Bekannt geworden sind die Todesfälle in der Tagespresse, durch den Einsatz von Beatmungsschläuchen für Kinder-Anestesie an der MHH-Hanover 1995. Restwassernester die im Biegebereich der Beatmungsschläuche im Schlauchinneren durch die Konstruktionsart der eingearbeiteten Stützspirale eingetrocknet und pulverisiert sind und später in die Lunge gelangten.

### 1.12 Stand der Technik

Der derzeitige technische Stand der Technick bei Vollautomatischen-Halbautomatischen und Manuellen Aufbreitungsverfahren zur Reinigung, Desinfektion und Neutralisation zur Anwendung für medizinisches Waschgut. Bei der nachstehenden Beschreibung der unterschiedlichen Systeme wird exemplarisch die Reinigung von flexiblen Endoskopen (thermolabiles Material) beschrieben.
Gleiches gilt anwendungsgemäß für anderes Wasch - Desinfektionsgut und Instrumente.

### 1.13 Vollautomatische Aufbereitungsverfahren

Bei der vollautomatischen Aufbereitung erfolgt die Reinigung und Desinfektion der endoskopischen Instrumente sowie das Nachspülen und Trocknen in einem hierzu konstruierten Automaten.
Verwendet werden chemische wie chemothermisch wirkende Desinfektionsmittel. Die Bauform ist im weitesten vergleichbar mit Haushalts-Geschirrspülmaschinen. Die Trocknung erfolgt über Heizungsspiralen unterhalb der Korbsysteme und durch Gebläse.
In der Waschkammer, sind Auszugskorbwagen angeordnet, diese haben Lagerungsaufnahmen für Endoskope mit Art waagerechten Aufnahme, wo das Spülgut leicht nach unten geneigt ist. Es erfolgen ein oder mehrere Flüßigkeits- und Druckluft-Verbindungsanschlüße zum Durchspülen der inneren Kanäle, sowie für einen Dichtigkeitstest für das Endoskop.

Es können bis zu drei flexible Endoskope übereinander im Korbsystem kreisförmig flüssigkeitsmäßig angeschlossen werden und vollautomatisch aufbereitet werden. In der Waschkammer sind oben und unten Sprühdüsen angeordnet. In einigen Ausführungen sind diese durch Wasserkraft selbstdrehende Spülarme mit Strahldüsen-Anordnung unterhalb bzw. oberhalb des Waschgutes angeordnet.
Die Waschkammer wird mit einer Frontbedienungsklapptür verschlossen, die Scharnieranordnung ist waagerecht. Über diese Klappenverschlußtür werden die Waschgut-Korbaufnahmewagen zur besseren Beschickung heraus- und hineingeschoben.
Durchschub und Durchladevarianten, bringen eine Arbeitsplatztrennung in Reine- Unreinezone und eine Trennung der Aufbereitungsräume.

### 1.14 Vollautomatisches Aufbereitungsverfahren mit Druckkammer

Der Verfahrensablauf unterscheidet sich gegen über dem vorstehenden, in der Form, das nur eine chemothermische Desinfektion zur Anwendung kommt.
Das Desinfektionsverfahren läuft vollautomatisch ab. Der Unterschied liegt im Reinigungsgut-und Aufnahmewagen für die flexiblen Endoskope. Diese ist mit Druckkammern ausgestattet. Das Bedienungsteil findet Aufnahme in der Druckkammer, der flexible Einführungsschlauch des wasserfesten Endoskopes wird in ein nach unten wendelartig geführtes Kunststoffrohr eingeführt. Druckkammer und flexibeles Kunststoffrohr übernehmen die Aufnahme des Endoskopes. Das vollautomatische Reinigungs- und Desinfektionverfahren läuft über die Druckkammer, in dem die Reinigungs- Desinfektionsmittel und die Nachspüllösungen ohne Übertragungsverbindungen zum Endoskop gestaltet sind. Zusätzliche Spülarme besprühen die Versorgungschläuche sowie das Zubehör der Endoskope.

### 1.15 Vollautomatische Desinfektion

Für Großkliniken und Zentralsterilisationen werden Durchlaufförderbandsysteme eingesetzt, mit einer chemothermischen Desinfektion. Die Transportkörbe werden mit dem Waschgut beschickt und über eine automatische Reinigungsfolge nach einem Fließband - Durchlaufverfahren gereinigt, desinfiziert und getrocknet.

### Erklärung: Desinfektion

Bei der chemischen Desinfektion werden nicht alle Mikroorganismen abgetötet.
Man unterscheidet high-level,
intermediatelevel und low-level Desinfektion:

Bei der high und intermediate-level Desinfektion;
werdem vegetive Bakterien einschließlich Myobaterium tuberculosis abgetötet, nicht jedoch Bakteriensporen.

Low-level-Desinfektion tötet alle vermehrungsfähigen Mikroorganismen ab, nicht jedoch Mykobakterien und Sporen.

Nicht kritische Oberflächen, der Instrumente, die intakte Haut berühren, aber nicht penetrieren, können mit intermediate-level oder low-level Desinfektion, desinfiziert werden.

Das ideale Desinfektionsmittel gibt es bisher nicht. Die Wahl stellt daher einen Kompromiß aus Toxizität, gesundheitlichen Nebenwirkungen, Breite der antimikobiellen Effektivität, Zeitdauer der erforderlichen Einwirkzeit und materialschonenden Effekten auf Beschichtungen, Kunststoff, Gummi, Linsen und Metalle dar.

### 1.16 Halbautomatische Aufbereitung

Bei der halbautomatischen Aufbereitung werden die Endoskope in Wannen, in der Regel aus Kunststoff vollständig getaucht, so daß alle Bauteile Flüssigkeitsmäßig mit Desinfektion- Neutralisationssmittel bedeckt sind.
Zum sparsameren Verbrauch der Desinfektionsmittel sind die Wannenerhöhungen -und Vertiefungen nach den Konturen der Endoskope geformt und haben ein Gefälle zum Ablauf. Die Befüllung kann sowohl von oben erfolgen, wie gleichermaßen durch Absperrorgane, die als Sperr- oder Wegeventile ausgebildet sind von unten gesteuert und gefüllt werden. Die Pumpen befinden sich unter dem Wannenkörper. Die Gerätekanäle werden an Umwälzpumpen angeschlossen und über eine einstellbare Zeitdauer mit den Medien durchspült.

### 1.17 Halbautomatische Aufbereitung

Wie vorstehend beschrieben gibt es eine weitere Ausführungsform durch Wannen zum Einlegen der flexiblen Endoskope, der flexible Einführungsschlauch wird in einen angesetzten Behälter oder Aufnahmerohr-Vorrichtung eingeführt.
Das Bedienungsteil und der Versorgungsschlauch des Endoskopes liegen im Wannenteil.

### 1.18 Manuelle Aufbereitungsverfahren

Nur der Ordnung halber vorgestellt; die gewöhliche manuelle Aufbereitung. Nach gründlichem mechanischen Reinigen der Außenteile und dem Durchbürsten der Kanalsysteme des Endoskopes, erfolgt ein Durchspülen der Kanäle zur Desinfektion. Nach alleinigem Waschen in einem Desinfektionsmittel-Wannenbad, werden die Kanäle per Hand, Spritze, Pumpe, luftfrei durchspült. Reinigungs-, Desinfektionsmittel und Leitungswasser bzw. Aqua dest wird verwendet. Es erfolgt keine Trocknung.

Ein Nachteil der manuellen Aufbereitung ist die Geruchsbelästigung durch die offene Wannenform und gesundheitliche Nebenwirkungen durch Infektionsallergien der Haut und Schleimhäute des Personals. Grundsätzlich müssen Schutzkittel, Schutzhandschuhe, Mund-Nasen-Schutz und Schutzbrille getragen werden.

### Erklärung: Weitere Desinfektionsvarfanten

Als **Sterilisation**
werden physikalische oder chemische Verfahren bezeichnet, die jegliche Mikroorganismen inclusive Sporen abtöten. Dieses kann durch Bestrahlen in Gamma oder Plasmasterilisatoren durch Gas in Form von Ethylenoxid und Formaldehyde geschehen. In Autoklaven erfolgt die Sterilisation durch Hitze oder Dampf.

**Chemieflüssigkeits-Kartuschensysteme** werden in wechselbaren Wannensystemen zur Reinigung, Desinfektion und Neutralisation in den USA eingesetzt. Den Verfahrensablauf kann man als Halb- Vollautomaisch wählen. Es handel sich um ein Einwegverfahren, zu jeder neuen Charge ist eine neue Chemiekartusche notwendig.

**Alkohol** tötet zwar Vieren ab, nicht jedoch vegetative Organismen, Sporen.
Die Verwendung von geringen Mengen Alkohol im Kanalsytem der Endoskope ist bekannt. Verbleibende geringe Mengen sind jedoch brennbar und explosiv. Daneben gibt es Materialunverträglichkeiten durch die Anlösung von Klebestellen.

**Enzymen,** Spezialreiniger mit organischen Zellstrukturen sind in der Klinischen Erprobung und erreichen nur eine verkürzte Reinigung. Auf den Einsatz einer nachfolgenden Desinfektion und Neutralisation kann nach heutigem Wissenstand nicht verzichtet werden.

### 2.0 Technische Aufgabe:

Die technisch gestellte Aufgabe, einer hohen hygienischen, einwandfreien überprüfbaren Qualität im automatischen Aufbereitungsverfahren für die hoch sensiblen Einsatzbereiche des Aufbreitungsgutes machte dem heutigem Wissensstand eine neue Erfinderische Konstruktion und Verfahrensablauf notwendig.

Die Hauptaufgabe der Vorrichtung besteht in der Verhinderung der Übertragung von schwerwiegenden Infektionen durch Mikroorganismen für nachfolgende Patienten, Arzte und Personal ist nur durch eine gründliche standardisierte automatische Aufbereitung des OP- und Klinkinstumentarium möglich.
Durch Mikroprozessorsteuerung mit permanenter Funktionskontrolle, aller angesteuerten Ein- und Ausgänge. Druck und Fehlerüberwachung sowie Temperatur und Dosierungsregelung werden durch unterschiedliche Programme, die auf die Waschgutart angepaßt sind, im Programmspeicher abrufbar hinterlegt.

Nur durch eine gründliche automatische zusätzliches Düsen-Bürsten Reinigungsystem ist es möglich eine Zielgerechte Reinigung des Außenmantels des Patienten-Einführungsschlauches zu gewährleisten. Die schnelle Koagulation von Proteinen wie Schleim, Sekret, Blut oder Stuhl erfordern ein umspülendes Düsen-Bürstensystem um Aldehyde-Alkohol bedingte feste Verunreinigungsreste und kristalline Ablagerungen rückstandslos zu entfernen.
So erfolgt eine intensivere Reinigungsbearbeitung des Außenmantels im Bereich des Instrumententeiles welche beim Patienten eingeführt war. Die unkritische Oberfläche des Versorgungsschlauches und des Bedienungsteil des Endoskopes werden einem Reinigungs-und Düsenspülverfahren unterzogen.

Anwenderart bedingte, lange flexible Instrumente die in Körperöffnungen eingeschoben werden haben verschiedene Innenkanäle.
Zum Fallbeispiel Endoskope; handelt es sich überwiegend um rundes Waschgut, im Bereich der Patientenanwendung.

Der Erfindung lag die Aufgabe zu Grunde, eine Vorrichtung zu schaffen, bei der das Desinfektionsmittel wiederverwendet wird. Dieses bedeutet eine Reduzierung des Desinfektionsmittelverbrauches der Kosten und der Abwasserbelastung. Umweltgerecht wird das chemische Desinfektionsmittel mengenmäßig über eine maschinelle Dosierung im Vorratsbehälter bereitgestellt.

Das Desinfektionsmittel wird pro Waschkammer getrennt im Kreislauf durch einen Vorratsbehälter geführt, so daß eine Trennung der Waschkammern für die Nutzerwünsche flexibel angepaßt werden kann.

Die chemischen Desinfektionsmittel lassen heute eine mehrfach Verwendung nach dem Grad der Verunreinigung zu.
Durch die Erfindergemäße Anwendung, wird die Desinfektionslösung gereinigt und mitschwimmende organische Schwebeteilchen werden dem Desinfektionsmittel im Spülgang permanent entzogen. So ist ein Desinfektionslösungswechsel, arbeitstäglich oder nach Benutzungszeiten möglich.
Somit ist das System sehr wirtschaftlich und sehr umweltfreundlich.

Durch Waschguttrennung ist eine hygienischere Vorreinigung möglich, in dem der automatische Programmablauf durch spezielle Sprühdüsen und Reinigungsverfahren verstärkt wird und die das Entfernen von angetrockneten Schleim, Sekreten, Blut oder Stuhl vereinfachen und so zu einer besseren und sicheren Desinfektion beitragen. Durch die verbesserte Qualität des erzielten Reinigungsergebnisses, die ein Aufbereitungsgut schonendes Verfahren sind, trägt eine höhere Sicherheit dazu bei, das eine Infektionsgefährdung minimiert oder ausgeschlossen wird.

Für jede Charge wird, frisches Reinigungsmittel und keimfreies Frischwasser verwendet um eine Rückverkeimung auszuschließen.
Leitungswasser wird über eine UV-Anlage keimfrei aufbereitet und in den VorZwischen und im Neutralisationsgang verwendet.

Das Waschgut wird nur oben gehalten oder in Vorrichtungen gehängt. Weitere Auflagepunkte wie Auflagebügel gibt es nicht. Eine Überdeckung wird vermieden. Eine nachteilhafte Reinigung durch Spülschatten wird vermieden. Anwendergemäß hängt das Endoskop und der Patienten-Einführungsschlauch und das innere Kanalsystem senkrecht ohne Knicke und Biegefalten. Dieses bewirkt eine sehr schnelle Reinigungs-Desinfektion-und Trocknungsszeit durch einen schnelleren Ablauf der ein- und aufgebrachten Flüssigkeiten. Durch den Einsatz von filtrierter Preßluft und trockener Warmluft wird ein sehr schneller senkrechter Abtrocknungsprozess, durch Schwerkraft sowohl innen wie außen von dem Waschgut erreicht.
Dieses ist eine erhebliche Verbesserung und trägt dadurch zur Verhinderung der Rekontamination und der Aufkeimung von Mikroorganismen bei.

Die Dichtungen der Türen mit ummanteltem Mangnetsystem sind leicht zu reinigen und können nachgestellt werden. Ein vorgezogenes Tropfblech sorgt dafür, das Feuchtigkeitsreste beim öffnen der Türen nicht auf den Fußboden gelangen, hierdurch wird eine Kontaminierung verhindert.

### 2.5 Offenbarung der Erfindung und deren Vorteile:

Nach der vorliegenden Erfindung, wird das Wasch- und Desinfektionsgut senkrecht gereinigt, die Aufnahmevorrichtung besteht aus durchspühlbarem Edelstahldrahtgitter. Die Erfindung erfüllt durch Ihre Bauform für längliches Waschgut die hygienischen Ansprüche, das die zu reinigenden und zu desinfizierenden medizinischen, technischen Geräte und Instrumente nur durch obere senkrechte Halterungen, freihängend ohne weitere Auflagen in den Waschkammern durch ein vollautomatisches Aufbereitungsverfahren gereinigt, desinifziert und neutralisiert werden.
Die erfindergemäße Ausführungsform, hat den Vorteil, daß das häufig runde flexible Waschgut, ( 70+72 ) knickfrei senkrecht frei hängt und keine Falten und Taschenknickstellen auf der Außenseite auftreten. Für die inneren Versorgungsleitungen für Flüssigkeiten und Luft in den Instrumenten und Geräten sowie für Schlauchmaterial hat diese Anwendung ebenfalls den gleichen Vorteil, der knickfreien verbesserten Reinigung und Desinfektion und der geringeren Feuchtigkeitsreste.

Durch ein zusätzliches Düsen-, Bürsten-Reinigungssystem wird der Außenmantel des Waschgutes, hier am Beispiel flexibles Endoskop, Patienten-Einführungsschlauch komplett umwaschen, so wird sichergestellt, das Verunreinigungsreste in den Faltenbereichen des Außenmantels rückstandslos entfernt werden.

Das Anwendungsverfahren hat die weiteren verbesserten Vorteile in der knickfreien Flüssigkeitsableitung sowohl außen wie innen bzw. in den Versorgungsleitungen und eine wesentlich schnellere Trocknung. Dieses bewirkt eine sehr schnelle Wiederverfügbarkeit des Aufbereitungsgutes. Eine Keimbildung wird durch filtrierte Druckluft und trockener Warmluft minimiert.

Der Erfindergemäße Vorteil ist durch die Trennung der Aufbereitungskammern erfolgt. Die freie zeitliche und unabhängig zur Verfügung stehenden Aufbereitungskammern gewährleisten eine ständige Einsatzmöglichkeit, ohne Wartezeiten. Dadurch erzielt der Nutzer eine schnellere Möglichkeit des Wiedereinsatzes des Aufbereitungsgutes.

Die Erfindung zeichnet sich durch ein einfaches Handling In der Bedienung und Beschickung der Waschkammern mit dem Waschgut aus. Durch die Waschkammergestaltung und die Baugruppen Instrumentenaufnahme ist eine universelle Instrumentenaufnahme(60) und der abnehmbare Kleinteilebehälterkorb (62) zur Aufnahme von Ventilen und Zubehör arbeitsplatzgerecht angeordnet.

Gleich, welche Baugröße die Bedienungsteile der flexiblen Endoskope(70) haben und in welcher Form und Gestaltungsart, sowie die Lage der Verbindungsanschlüsse am Versorgungsschlauch des Endoskopes vorhanden sind, eine vorhandene Instrumentenaufnahme (60) mit variabeler Höhenverstellung (61) und eine Universalaufnahme für den Versorgungsschlauchstecker(75) nehmen alle Bauausführungen auf.

Es erfolgt pro Aufbereitungskammer eine getrennte Wiederverwendung vom chemischen Desinfektionsmittel durch drucklose Vorratsbehälter die im Aggregatbereich angeordnet sind. Die umweltfreundliche Wiederverwendung ermöglicht diesem Verfahren eine hohe Wirtschaftlichkeit.

Die chemischen Desinfektionsmittel lassen eine Mehrfachnutzung bei gleichem Aufbereitungsgut zu. Die Verwendungsdauer ist abhängig durch den Verschmutzungsgrad und der Benutzungszeit. Bei geringer Auslastung ist nach Angaben der Desinfektionsmittelhersteller eine Verwendbarkeit bis zu einer Woche gegeben. Bei hoher Frequenz sollte arbeitstäglich, gewechselt werden.

Mitschwimmende organische Schwebeteilchen werden durch eine Turbinenbauart dem Desinfektionsmittel im Spülgang permanet entzogen. Somit verlängert sich die Einsatzzeit der Wiederverwendbarkeit des Desinfektionsmittels.

### 3.0 Bevorzugte Ausführungsform der Erfindung

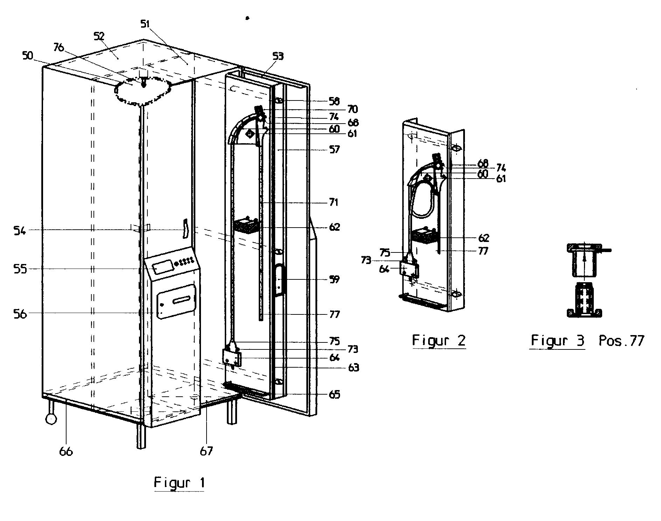

Kammerschrankbauform mit einer, zwei oder mehreren Türen ( 53 ) und Korbauszüge, die durch Teleskope ein-und ausfahrbar sind. Für das Aufbereitungsverfahren nach Zeichnung, Figur 2, kann auch unterhalb der Aufbereitungskammern ein Schrankraum für Chemie etc. angeordnet sein. Die Instrumentenaufnahme ist für das jeweilige Waschgut leicht wechsel- und austauschbar.
Fallbeispiel nach Zeichnung, Anwendung für flexible Endoskope.

Bauform für parallel Durchlademöglichkeit, Reine - Unreine Seite, Aggregatanordnung, hinten, seitlich oder in der Mitte.

Das chemische Desinfektionsmittel, oder chemothermische Desinfektionsmittel wird mengenmäßig über eine maschinelle Dosierung im Vorratsbehälter mit einer jeweiligen Trennung für die Anwendungsbereiche bereitsgestellt und im Spülgang Desinfektion im Kreislauf geführt.
Es erfolgt beim Einsatz von chemischen Desinfektionsmittel eine Wiederverwendung, je nach Benutzungsfrequenz, oder Betriebsstunden ist eine umweltgerechte Nutzung je nach Fabrikat des Desinfektionsmittels bis zu einer Woche möglich.

### 3.1 Aggregateanordnung

Durch die unterschiedliche Wasserangebotsqualität in den Kliniken kann die Wasserversorgung und die damit verbundene Aufarbeitungsmöglichkeiten jeweils angepasst werden. Ein Direktanschluß ans Wasserleitungsnetz ist vorgesehen, der Einsatz von geprüften und zugelassenen Sperrventilen, Druckminderer, Entkalker, Inonentauscher und UV-Licht Wasserdesinfektion sorgt für gleiche, desinfizierte Wasserqualität. Die weiteren Baugruppen Dosiereinrichtungen, Desinfektions-Vorratsbehälter, Steuerventile, Pumpen und Kompressoren Warmluftgebläse, Abwasseranschlüsse u.a.m. werden mit Verbindungen in die Aufbereitungskammern auf der Rückseite (52) der Maschine eingebaut.

Pro Aufbereitungsraum steuert je eine elektrische programmierbare Steuerung unabhängig voneinander die einzelnen Aufbereitungskammern (50+51)

Die Aggregate und Baugruppen und die programmierbare Maschinensteuerung werden als Handelskaufteile einzeln, als Baugruppe oder komplett vormontiert eingesetzt. Die notwendigen Zulassungsprüfungen sind durchzuführen.

Die elektrische Bedienung (55) der einzelnen Waschkammern erfolgt durch ein Bedienungspult welches vor den Türen angeordenet ist. Die elektrische Bedienungsfläche ist schräg gestaltet, um eine bessere Funktion und Kontrolle für den Nutzer zu ermöglichen.
Unterhalb des Bedienungspultes(56) kann ein Protokolldrucker das vollautomatische Aufbereitungsverfahren pro Aufbereitungskammer dokumentieren, den Programmablauf- Zeit und Art, Datum, Uhrzeit, Chargenlaufzeit und eventuelle Störmeldungen. Hierzu ist unterhalb des elektrischen Bedienungspultes eine Zugangsklappe oder ein senkrechtes Verschlußblech angeordnet.

### 4.0 Gewerbliche Anwendbarkeit:

Die Anwendung kann erfolgen im Einsatz für die Humanmedizin sowie in der Veterinärmedizin.
Dieses können sein; Universitätskliniken, Krankenhäuser, Kliniken, Facharztzentren, niedergelassene Internisten und Fachärzte.
Gleichfalls ist die Anwendung für Industriewaschgut möglich.

### 4.5 Zeichnungserklärung

Der Erfindergegenstand wird im Ausführungsbeispiel mit einem flexiblen Endoskop in Zeichnung, Figur (1) erklärt.

In der Zeichnung, Figur (2) ist eine Anwenderart für flexible Endoskope der Bauarten; Sigmoidoskope, Bronchoskope, Choloedochoskope, Nasopharingoskope, Hysteroskope u.a.m. dargestellt. Diese Instrumente haben ein kurzes Patienten-Einführungsteil bis ca. 0,70 m ohne Bedienungsteil.

Figur (3) zeigt die Funktionsweise des zusätzlichen Reinigungsverfahrens für Anwendung auf dem Patienten-Einführungsteil

### Liste der Bezugszeichen:

- 50: Aufbereitungskammer (1)
- 51: Aufbereitungskammer (2)
- 52: Aggregatanordnung
- 53: Tür
- 54: Türverriegelung
- 55: Elekt. Bedienung
- 56: E. -Service, Drucker
- 57: Korbauszug mit Instrumentenaufnahmevorrichtung
- 58: Teleskopauszüge
- 59: Korbauszug-Handgriff
- 60: Instrumentenaufnahme
- 61: Variable Höhenverstellung
- 62: Abnehmbarer Kleinteilebehälter
- 63: Rohrversorgungsleitung
- 64: Aufnahme f. Versorgungsschlauch
- 65: Auffangtropfblech
- 66: Auffangwanne/Notüberlaufw.
- 67: Ablauf mit losem Siebeinsatz
- 68: Kupplungsverbindungen z. Endoskop
- 70: Flexibles Endoskop, Bedienteil
- 71: Einführungsteil-Patienten
- 72: Endoskop-Versorgungsschlauch
- 73: Versorgungsanschlüsse für die inneren Kanäle u. Drucktest
- 74: Versorgungsanschlüsse für die inneren Kanäle
- 75: Versorgungsschlauchstecker
- 76: Sprühdüsen
- 77: Zusätzliches Düsen/Reinigungssystem

## Patentansprüche

1. Gekennzeichnet durch folgende Merkmale:
Das Waschgut, flexibles Endoskop, wird senkrecht frei über die Endoskopaufnahme (60) positioniert. Eine variable Höhenverstellung,(61) manuell, durch E.-Getriebemotor oder auf pneumatik Wege, sorgt für ein Arbeitsökonmisches einfaches Handling der Beschickung und der Entnahme des Waschgutes.

2. Die Anzahl der Aufbereitungskammern (50+ 51) ist nach Anwenderart und Bedarf beliebig in einer, zwei oder X Aufbereitungskammern in Schrankbauform herstellbar, Türenanordnung (53) senkrecht.
Türen-Varianten, allseits, auch als Durchlademodell möglich.
Variation und Größe der Aufbereitungskammer (50+51)und deren Ausstattung ist für den Kundenkreis, nutzungsgerecht anpaßbar.

3. Korbauszug mit Instrumentenaufnahme(57) wie Bauform gemäß beschrieben, jedoch auch variierent als Korbauszuge mit je zwei, oder mehr Teleskopauszügen (58)
Die Höhe der Aufbereitungskammern richet sich nach der Länge vom EndoskopEinführungsteil (71) des Patienten und kann bis Deckenhöhe verwirklicht werden.

4. Die Sprühdüsenanordnung ist variabel wählbar, je nach Waschgut und sorgt für eine ausreichende Flüssigkeitsbenetzung und Reinigung des Endoskopes (70+72) Der Patienten-Einführungsteil (71) wird über ein zusätzliches Düsen-, Bürsten-,Reinigungssystem (77) maschinell automatisch bearbeitet.
Das Reinigungssystem (77) besteht aus einem Turbinen-Antrieb, der durch die jeweilige Flüssigkeit und Druckluft angetrieben wird. Der Turbinen-Ausgang ist in der Bauweise gestaltet, das Ringdüsen das jeweilige Medium, gezielt auf das Aufbereitungsgut führen. Feine Innenbürsten unterstützen den Reinigungsvorgang. Durch ein einfaches schnelles Bausatzsystem erfolgt die Auswechselung des Bürsten- und Düsen- Innenteiles auf die jeweils notwendigen Innendurchmesser. Das Düsen-,Bürsten-,Reinigungssytem verhindert durch Ihre Bauart, das es zu einem verschleudern von Schmutzpartikeln auf andere Instrumententeile kommt. Der Reinigungsvorgang erfolgt in der Ringdüse, die nach innen gerichtet sind. Zusätzlich zum Reinigungsvorgang -Düsen- übernimmt eine feine Bürstenanordnung die Führung des Patienten-Einführungsschlauch (71) und eine zusätzliche Reinigung. So ist gewährleistet daß die Flüssigkeiten nur senkrecht abgeleitet werden. Der Bürstenbesatz ist so gewählt, das es zu keinem festsetzen von Schmutzpartikel kommen kann. Dieses wird durch die Strahlrichtung der Düsen und der 360 ° Drehungen verhindert. Im Korbauszug (57) wird das Reinigungssystem (77) geführt und wird durch einen Antrieb linear senkrecht auf und ab, bewegt. Die Reinigungslänge wird variabel eingestellt.

5. Das innere Kanalsystem des Endoskopes wird mit gefilterter Druckluft trockengeblasen, der Außenmantel des Einführungsteil (71) und der Versorgungsschlauch werden über zusätzliche Düsen (77) mit Warmluft getrocknet.

6. Der Korbauszug mit Instrumentenaufnahme(57) kann für flexible Endoskope, der Anwendungsarten, Bonchoskopie, Hysterunskopie u.a. so gestaltet sein, das der Versorgungsschlauch vom Endoskop, wie in Figur 2 dargestellt, um die Bauhöhe der Aufbereitungskammern zu verkürzen, ein, oder zweimal als Schlaufe positioniert wird. Es erfolgt keine Überdeckung.

7. Der Korbauszug(57) ist durch den Einsatz von Rundrohrmaterial (63) so gestaltet, daß die Rohre gleichzeitig zum Transport von flüssigen Chemiemittel und von gefilterter Luft zu Versorgungsanschlüsse (73+74) genutzt werden.
An der Vorderseite ist ein (59) Handgriff intregriert, der eine Verriegelung aufweist. Diese sichert den Teleskopauszug in der Form, das dieser im Aufbereitungsgang fest in Position gehalten wird.
Mit flexiblen Versorgungsschläuchen und Kupplungsverbindungen(68) erfolgt die Verbindung für die Versorgung der inneren Kanäle mit dem flüssigem Medium, sowie für den Transport der Durckluft(74)

8. Zur Versorgung der rückwärtigen Sprühdüsen die sich im Korbauszug (57) befinden (76) und des zusätzlichen Düsen und Reinigungssystem wird gleichfalls das Gitterkorbmaterial als Rohrleitungsnetz gefertigt um lange Verbindungs-Schlauchwege zu vermeiden.
Das Rohrleitungsnetz (63) wird ebenfalls zur Versorgung der Sprühdüsenanordnung (76+77) verwendet.

9. An der Unterseite des Korbauszuges ist ein Auffangtropfblech (65) intregiert, dieses verhindert Verschmutzung und Kontamination vor der Maschine. Beim Einschieben des Teleskopauszuges klappt sich das Auffangtropfblech senkrecht zurück und wird über angebaute Düsen mitgespült.

10. Unterhalb der Waschkammern ist eine Art Auffangwanne (66) ausgebildet, die sich unterhalb der Türen befindet, das beim Öffnen der Türen (53) , das in dem Türdichtungsbereich eingelagerte Restflüssigkeit nicht auf den Fußboden gelangt. Die gleiche Funktion wird für den rückwärtigen Aggregateraum als Notüberl auf genutzt.

11. Im Ablauf ist ein herausnehmbarer Siebeinsatz integriert, der die Aufgabe hat, das grober Schmutz, Kleinteile aus dem Instrumentarium etc. nicht mitschwimmen und in den Pumpenkreislauf gelangen.
